# EUROPEAN PATENT APPLICATION

(11) **EP 1 553 082 A1**
(43) Date of publication of application: **13.07.2005**
(21) Application number: 03760919.5
(22) Date of filing: 20.06.2003
(51) Int. Cl.: C07C 303/26, C07C 309/65, C07J 31/00

(54) **PROCESS FOR PRODUCTION OF VINYL PERFLUOROALKANESULFONATE DERIVATIVES**

(30) Priority: 20.06.2002 JP 2002179616
(71) Applicant: KYOWA HAKKO KOGYO CO., LTD., Chiyoda-ku, Tokyo 100-8185 (JP)
(72) Inventor: KATO, Sachiko c/o Head Office Kyowa Hakko Kogyo Co, Chiyoda-ku, Tokyo 100-8185 (JP); CHUJO, Iwao c/o Sakai Research Lab. Kyowa Kogyo Co, Sakai-shi, Osaka 590-8554 (JP); SUZUKI, Koji c/o Sakai Research Lab., Sakai-shi, Osaka 590-8554 (JP)
(74) Representative: Tanner, James Percival
(86) International application number: PCT/JP2003/007859
(87) International publication number: WO 2004/000795

(57) **Abstract**

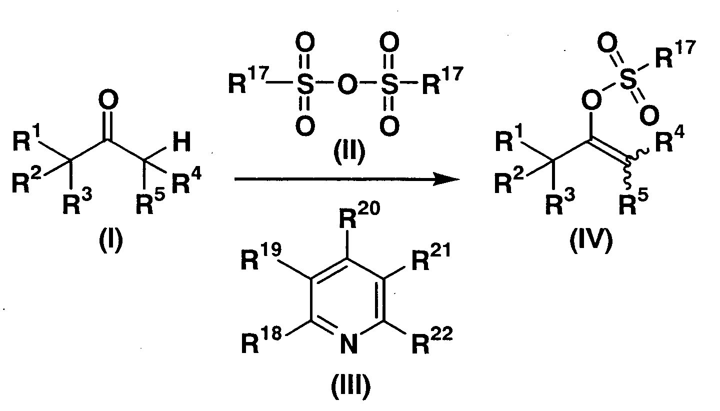

(wherein R¹, R², R³, R⁴ and R⁵ may be the same or different and each represents a hydrogen atom, substituted or unsubstituted lower alkyl, substituted or unsubstituted lower alkoxy, or the like; R¹⁷ represents trifluoromethyl or the like; R¹⁸, R¹⁹, R²⁰, R²¹ and R²² may be the same or different and each represents a hydrogen atom, substituted or unsubstituted lower alkyl, or the like).

An easy and simple process for producing a vinyl perfluoroalkanesulfonate derivative represented by general formula (IV) which is a useful intermediate in synthesis of medicines, natural products, or the like, at a low cost, is provided. The process is **characterized by**, for example, reacting a carbonyl compound represented by general formula (I) described above with a perfluoroalkanesulfonic anhydride represented by general formula (II) described above in the presence of a pyridine derivative represented by general formula (III) described above in an amount of 0.1 to 1.0 equivalent to the perfluoroalkanesulfonic anhydride.

## Description

### Technical Field

The present invention relates to processes for producing vinyl perfluoroalkanesulfonate derivatives which are useful as intermediates in synthesis of medicines, natural products, or the like.

### Background Art

Vinyl perfluoroalkanesulfonate derivatives are broadly used as useful intermediates in synthesis of medicines and natural products (see, for example, Synthesis, p. 735 (1993); Tetrahedron Lett., vol. 23, p. 117 (1982); ibid., vol. 24, p. 979 (1983); J. Org. Chem., vol. 56, p. 3486 (1991); ibid., vol. 57, p. 976 (1992); ibid., vol. 63, p. 4135 (1998)), and their inexpensive and simple manufacturing processes are desired.

It is known that a vinyl perfluoroalkanesulfonate derivative is prepared by reacting a perfluoroalkanesulfonic anhydride with a carbonyl compound in the presence of a base (Synthesis, p. 735 (1993)). This process of reacting the perfluoroalkanesulfonic anhydride with the carbonyl compound such as ketone or aldehyde generates perfluoroalkanesulfonic acid, which is a very strong acid, and it is problem that perfluoroalkanesulfonic acid generates by-products such as an aldol condensation product.

To inhibit the undesired reactions, a base must be added. A known method for inhibiting the undesired reactions includes steps of dissolving the carbonyl compound in a solvent, i.e. pentene, chloroform, methylene chloride, or carbon tetrachloride; adding a base such as pyridine or triethylamine in excess to trifluoromethanesulfonic anhydride; and then adding trifluoromethanesulfonic anhydride (Org. Synth., vol. 54, p. 79 (1974) and Synthesis, p. 85 (1982)). However, in this method, a salt is formed from the base with trifluoromethanesulfonic anhydride. As a result, vinyl trifluoromethanesulfonate derivative is formed in a low yield, and purification is difficult due to production of tarry materials.

As means for resolving above problems, a method of using 2,6-di-tert-butyl-4-methylpyridine as the base and reacting trifluoromethanesulfonic anhydride with a carbonyl compound is reported (J. Am. Chem. Soc., vol. 111, p. 8320 (1989); Synthesis, p. 283 (1980); ibid., p. 49 (1987); ibid., p. 735 (1993)). 2,6-Di-*tert*-butyl-4-methylpyridine specifically and efficiently reacts with trifluoromethanesulfonic acid to form a salt and does not form a salt with trifluoromethanesulfonic anhydride due to the steric factor. As a result, the production of by-products such as the aldol condensation product is inhibited and the yield of the vinyl trifluoromethanesulfonate derivative is improved.

2,6-Di-tert-butyl-4-methylpyridine is useful for synthesis in a laboratory scale, but inappropriate for use in an industrial scale because of the following reasons: (1) It is difficult to obtain in large quantity; (2) It is expensive; and (3) It is difficult to remove from the reaction system.

Another method for producing a vinyl trifluoromethanesulfonate derivative is known (Synthetic Communications, vol. 29, p. 409 (1999)). According to the method, the vinyl trifluoromethanesulfonate derivative is produced by reacting trifluoromethanesulfonic anhydride with cyclohexanone in the presence of potassium carbonate as a base, which is available in large quantity at a low cost and is readily removable from the reaction system. However, it is not a sufficient method as the synthesis in a large scale because of the low yield of vinyl trifluoromethanesulfonate derivative.

### Disclosure of Invention

An object of the present invention is to provide a simple and easy process for producing a vinyl perfluoroalkanesulfonate derivative useful as an intermediate in synthesis of medicines, natural products, or the like, in a large scale at a low cost by using reagents that are inexpensive and readily available in large quantity.

The present invention relates to the following aspects (1) to (17):
(1) A process for producing a vinyl perfluoroalkanesulfonate derivative represented by general formula (IV): 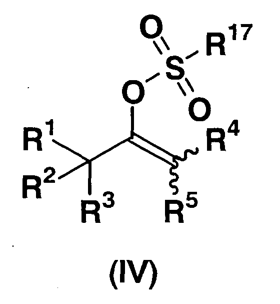 (wherein R¹, R², R³, R⁴ and R⁵ may be the same or different and each represents a hydrogen atom, substituted or unsubstituted lower alkyl, substituted or unsubstituted lower alkoxy, substituted or unsubstituted lower alkoxycarbonyl, substituted or unsubstituted lower alkanoyl, substituted or unsubstituted lower alkanoyloxy, substituted or unsubstituted lower alkenyl, substituted or unsubstituted lower alkynyl, substituted or unsubstituted lower alkadienyl, substituted or unsubstituted cycloalkyl, substituted or unsubstituted cycloalkenyl, substituted or unsubstituted cycloalkynyl, substituted or unsubstituted cycloalkadienyl, substituted or unsubstituted aralkyl, substituted or unsubstituted aralkyloxy, substituted or unsubstituted aralkyloxycarbonyl, substituted or unsubstituted aryl, substituted or unsubstituted aryloxy, substituted or unsubstituted aryloxycarbonyl, a substituted or unsubstituted heterocyclic group, nitro, nitroso, halogen, carboxy, -S(O)ₙR⁶ (wherein n represents 0 or 1, and R⁶ represents substituted or unsubstituted lower alkyl, substituted or unsubstituted aralkyl, or substituted or unsubstituted aryl group), -P(O)ₘR^{6a}R^{6b} (wherein m represents 0 or 1, and R^{6a} and R^{6b} may be the same or different and each has the same meaning as R⁶ defined above), or -NR⁷R⁸ [wherein R⁷ and R⁸ may be the same or different and each represents a hydrogen atom, substituted or unsubstituted lower alkyl, substituted or unsubstituted aralkyl, substituted or unsubstituted aryl, substituted or unsubstituted lower alkoxycarbonyl, substituted or unsubstituted lower alkanoyl, substituted or unsubstituted lower alkanoyloxy, -CONR^{6c}R^{6d} (wherein R^{6c} and R^{6d} may be the same or different and each has the same meaning as R⁶ defined above), or -SO₂R^{6e} (wherein R^{6e} has the same meaning as R⁶ defined above)];
   R¹ and R² are combined together with the adjacent carbon atom thereto to form R⁹ (wherein R⁹ represents substituted or unsubstituted cycloalkyl, substituted or unsubstituted cycloalkenyl, substituted or unsubstituted cycloalkynyl, substituted or unsubstituted cycloalkadienyl, or a substituted or unsubstituted heterocyclic group);
   R⁴ and R⁵ are combined together with the adjacent carbon atom thereto to form R¹⁰ (wherein R¹⁰ has the same meaning as R⁹ defined above);
   R¹, R² and R³ are combined together with the adjacent carbon atom thereto to form R¹¹ (wherein R¹¹ represents substituted or unsubstituted aryl, or a substituted or unsubstituted heterocyclic group);
   R¹ and R⁴ are combined together with the two carbon atoms which are adjacent to R¹ or R⁴, respectively, and the carbon atom between these two carbon atoms to form a substituted or unsubstituted carbocycle, or a substituted or unsubstituted aliphatic heterocycle; or
   R¹, R², R³, R⁴ and R⁵ are combined together with the two carbon atoms which are adjacent to R¹, R², R³, R⁴ or R⁵, respectively, and the carbon atom between these two carbon atoms to form a substituted or unsubstituted carbocycle, a substituted or unsubstituted aliphatic heterocycle, or a substituted or unsubstituted condensed ring, and
   R¹⁷ represents a fluorine atom or perfluoroalkyl)
   which comprises reacting a carbonyl compound represented by general formula (I): 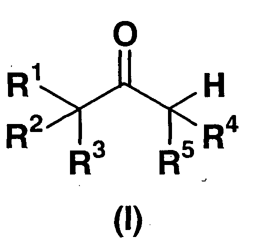 (wherein R¹, R², R³, R⁴ and R⁵ have the same meanings as defined above, respectively)
   with a perfluoroalkanesulfonic anhydride represented by general formula (II): 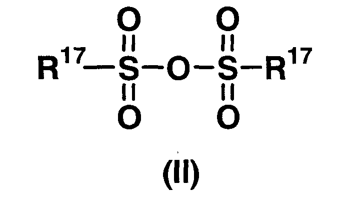 (wherein R¹⁷ has the same meaning as defined above)
   in the presence of a pyridine derivative represented by general formula (III) in an amount of 0.1 to 1.0 equivalent to the perfluoroalkanesulfonic anhydride: 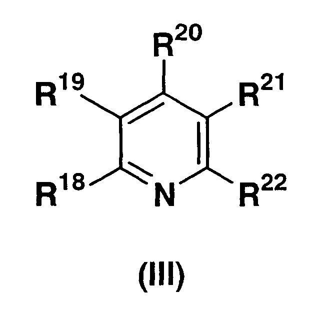 (wherein R¹⁸, R¹⁹, R²⁰, R²¹ and R²² may be the same or different and each represents a hydrogen atom, halogen, substituted or unsubstituted lower alkyl, or substituted or unsubstituted lower alkoxy; with the proviso that when R¹⁸ and R²² are *tert*-butyl, and R¹⁹ and R²¹ are hydrogen atoms, R²⁰ is not methyl).
(2) A process for producing a vinyl perfluoroalkanesulfonate derivative represented by general formula (IV): 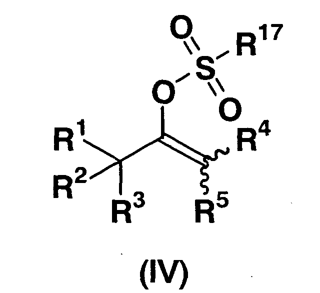 (wherein R¹, R², R³, R⁴, R⁵ and R¹⁷ have the same meanings as defined above, respectively) which comprises adding a carbonyl compound represented by general formula (I): 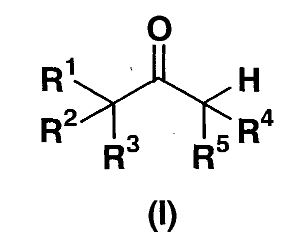 (wherein R¹, R², R³, R⁴ and R⁵ have the same meanings as defined above, respectively)
   to a suspension or a solution containing a perfluoroalkanesulfonic anhydride represented by general formula (II): 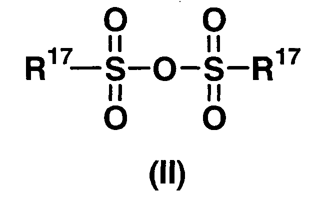 (wherein R¹⁷ has the same meaning as defined above)
   and a pyridine derivative represented by general formula (III) in an amount of 0.1 to 1.0 equivalent to the perfluoroalkanesulfonic anhydride: 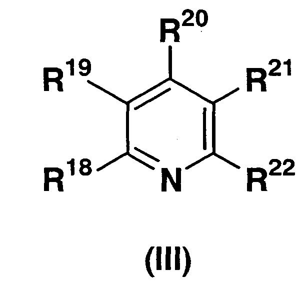 (wherein R¹⁸, R¹⁹, R²⁰, R²¹ and R²² have the same meanings as defined above, respectively), and wherein
   when the content of the pyridine derivative represented by general formula (III) in the suspension or the solution is 1.0 equivalent to the perfluoroalkanesulfonic anhydride represented by general formula (II), the perfluoroalkanesulfonic anhydride represented by general formula (II), water, an acid, or an acid anhydride is further added for the reaction.
(3) A process for producing a vinyl perfluoroalkanesulfonate derivative represented by general formula (IV): 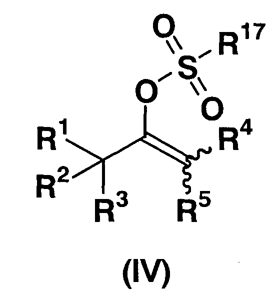 (wherein R¹, R², R³, R⁴, R⁵ and R¹⁷ have the same meanings as defined above, respectively)
   which comprises reacting a carbonyl compound represented by general formula (I): 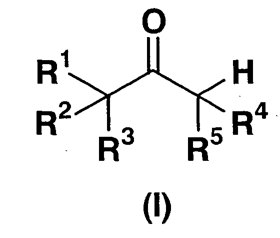 (wherein R¹, R², R³, R⁴ and R⁵ have the same meanings as defined above, respectively)
   with a 1-(perfluoroalkanesulfonyl)pyridinium perfluoroalkanesulfonate represented by general formula (V): 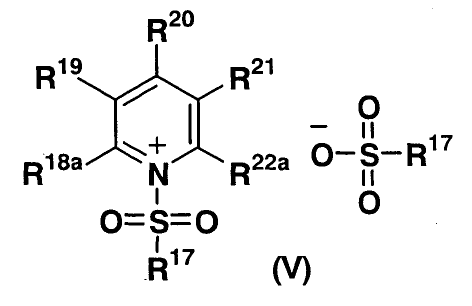 (wherein R¹⁹, R²⁰ and R²¹ have the same meanings as defined above, respectively, and R^{18a} and R^{22a} may be the same or different and each represents a hydrogen atom, methyl, ethyl, *n*-propyl, isopropyl, methoxy, ethoxy, *n*-propyloxy, or isopropyloxy).
(4) A process for producing a vinyl perfluoroalkanesulfonate derivative represented by general formula (IV): 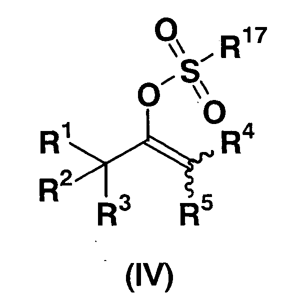 (wherein R¹, R², R³, R⁴, R⁵ and R¹⁷ have the same meanings as defined above, respectively)
   which comprises preparing a 1-(perfluoroalkanesulfonyl)pyridinium perfluoroalkanesulfonate represented by general formula (V): 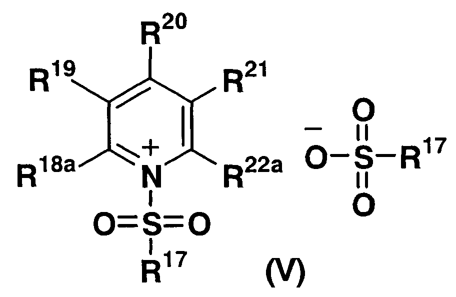 (wherein R^{18a}, R¹⁹, R²⁰, R²¹ and R^{22a} have the same meanings as defined above, respectively)
   from a perfluoroalkanesulfonic anhydride represented by general formula (II): 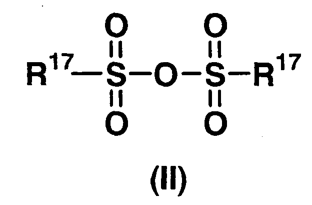 (wherein R¹⁷ has the same meanings as defined above)
   and a pyridine derivative represented by general formula (IIIa): 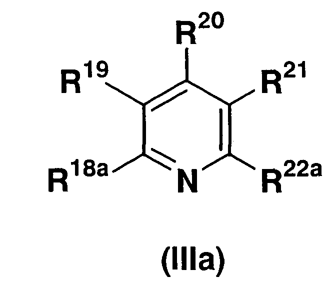 (wherein R^{18a}, R¹⁹, R²⁰, R²¹ and R^{22a} have the same meanings as defined above, respectively);
   and then reacting the resulting 1-(perfluoroalkanesulfonyl)pyridinium perfluoroalkanesulfonate with a carbonyl compound represented by general formula (I): 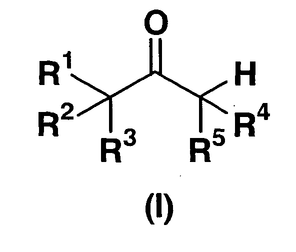 (wherein R¹, R², R³, R⁴, and R⁵ have the same meanings as defined above, respectively).
(5) The process according to (1) or (2), wherein R¹⁸ and R²² may be the same or different and each represents a hydrogen atom, methyl, ethyl, *n*-propyl, isopropyl, methoxy, ethoxy, *n*-propyloxy, or isopropyloxy.
(6) The process according to (1) or (2), wherein R¹⁸ and R²² may be the same or different and each represents a hydrogen atom, halogen, or methyl.
(7) The process according to any one of (1) to (6), wherein R¹⁹ and R²¹ represent a hydrogen atom.
(8) The process according to (7), wherein R²⁰ represents a hydrogen atom or methyl.
(9) The process according to (1) or (2), wherein R¹⁸, R¹⁹, R²⁰, R²¹, and R²² represent a hydrogen atom.
(10) The process according to (3) or (4), wherein R^{18a}, R¹⁹, R²⁰, R²¹, and R^{22a} represent a hydrogen atom.
(11) The process according to any one of (1) to (10), wherein the perfluoroalkanesulfonic anhydride represented by general formula (II), water, an acid, or an acid anhydride is further added during the reaction of the perfluoroalkanesulfonic anhydride represented by general formula (II): 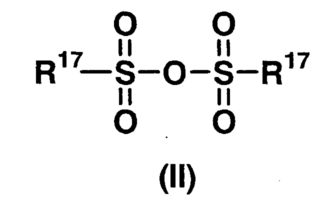 (wherein R¹⁷ has the same meaning as defined above).
(12) The process according to any one of (1) to (11), wherein R¹⁷ represents a fluorine atom, trifluoromethyl, or nonafluoro-*n*-butyl.
(13) The process according to any one of (1) to (11), wherein R¹⁷ represents a fluorine atom or trifluoromethyl.
(14) The process for producing a vinyl perfluoroalkanesulfonate derivative according to any one of (1) to (13), wherein at least one selected from the group consisting of methylene chloride, toluene, chlorobenzene, trifluorotoluene, and dichlorobenzene is used as the solvent.
(15) The process for producing a vinyl perfluoroalkanesulfonate derivative according to any one of (1) to (14), wherein R¹ and R⁴ are combined together with the two carbon atoms which are adjacent to R¹ or R⁴, respectively, and the carbon atom between these two carbon atoms to form a substituted or unsubstituted carbocycle, or a substituted or unsubstituted aliphatic heterocycle; or R¹, R², R³, R⁴ and R⁵ are combined together with the two carbon atoms which are adjacent to R¹, R², R³, R⁴ or R⁵, respectively, and the carbon atom between these two carbon atoms to form a substituted or unsubstituted carbocycle, a substituted or unsubstituted aliphatic heterocycle, or a substituted or unsubstituted condensed ring.
(16) The process for producing a vinyl perfluoroalkanesulfonate derivative according to any one of (1) to (14), wherein R¹, R², R³, R⁴ and R⁵ are combined together with the two carbon atoms which are adjacent to R¹, R², R³, R⁴ or R⁵, respectively, and the carbon atom between these two carbon atoms to form a substituted or unsubstituted carbocycle.
(17) The process for producing a vinyl perfluoroalkanesulfonate derivative according to any one of (1) to (14), wherein R¹, R², R³, R⁴ and R⁵ are combined together with the two carbon atoms which are adjacent to R¹, R², R³, R⁴ or R⁵, respectively, and the carbon atom between these two carbon atoms to form a substituted or unsubstituted condensed ring.

Hereinafter, compounds represented by general formulae (I), (II), (III), (IIIa), (IV), and (V) are referred to as Compounds (I), (II), (III), (IIIa), (IV), and (V), respectively. Compounds represented by other formulae will be referred to in the same manner.

In the definitions of groups in general formulae (I), (II), (III), (IIIa), (IV), and (V):
(a) Examples of alkyl moieties of the lower alkyl, lower alkoxy, lower alkoxycarbonyl, lower alkanoyl, lower alkanoyloxy, and perfluoroalkyl include linear or branched alkyl having 1 to 9 carbon atoms, i.e. methyl, ethyl, propyl, isopropyl, butyl, isobutyl, sec-butyl, tert-butyl, pentyl, isopentyl, neopentyl, hexyl, heptyl, octyl, isooctyl, nonyl, and decyl;
(b) Examples of the lower alkenyl include linear or branched alkenyl having 2 to 6 carbon atoms, i.e. vinyl, allyl, 1-propenyl, methacryl, crotyl, 1-butenyl, 3-butenyl, 2-pentenyl, 4-pentenyl, 2-hexenyl, and 5-hexenyl;
(c) Examples of the lower alkynyl include linear or branched alkynyl having 2 to 6 carbon atoms, i.e. ethynyl, propynyl, butynyl, pentynyl, and hexynyl;
(d) Examples of the lower alkadienyl include linear or branched alkadienyl having 2 to 8 carbon atoms, i.e. 1,3-butadienyl, 1,3-pentadienyl, 2,4-pentadienyl, and 1,5-dimethyl-1,4-hexadienyl;
(e) Examples of the cycloalkyl include cycloalkyl having 3 to 8 carbon atoms, i.e. cyclopropyl, cyclobutyl, cyclopentyl, cyclohexyl, cycloheptyl, and cyclooctyl;
(f) Examples of the cycloalkenyl include cycloalkenyl having 4 to 8 carbon atoms, i.e. cyclobutenyl, cyclopentenyl, cyclohexenyl, cycloheptenyl, and cyclooctenyl;
(g) Examples of the cycloalkynyl include cycloalkynyl having 4 to 8 carbon atoms, i.e. cyclobutynyl, cyclopentynyl, cyclohexynyl, cycloheptynyl, and cyclooctynyl;
(h) Examples of the cycloalkadienyl include cycloalkadienyl having 4 to 8 carbon atoms, i.e. cyclobutadienyl, cyclopentadienyl, cyclohexadienyl, cycloheptadienyl, and cyclooctadienyl;
(i) Alkylene moieties of the aralkyl, aralkyloxy, and aralkyloxycarbonyl are each synonymous with lower alkyl from which one hydrogen atom is removed;
(j) Examples of aryl moieties of the aryl, aryloxy, aryloxycarbonyl, aralkyl, aralkyloxy, and aralkyloxycarbonyl include aryl having 6 to 14 carbon atoms, i.e. phenyl, naphthyl, and anthryl; and
(k) Examples of the heterocyclic group include an aromatic heterocyclic and aliphatic heterocyclic group.
   Examples of the aromatic heterocyclic group include a five- or six-membered monocyclic aromatic heterocyclic group containing at least one atom selected from a nitrogen atom, an oxygen atom, and a sulfur atom; and a bicyclic or tricyclic condensed aromatic heterocyclic group which is formed by condensation of three- to eight-membered rings and contains at least one atom selected from a nitrogen atom, an oxygen atom, and a sulfur atom. In particular, pyridyl, pyrazinyl, pyrimidinyl, pyridazinyl, benzimidazolyl, 2-oxobenzimidazolyl, benzotriazolyl, benzofuryl, benzothienyl, purinyl, benzoxazolyl, benzothiazolyl, benzodioxolyl, indazolyl, indolyl, isoindolyl, purinyl, quinolyl, isoquinolyl, phthalazinyl, naphthyridinyl, quinoxalinyl, pyrrolyl, pyrazolyl, quinazolinyl, cinnolinyl, triazolyl, tetrazolyl, imidazolyl, oxazolyl, isooxazolyl, thiazolyl, isothiazolyl, thienyl, furyl, and the like are included.
   Examples of the aliphatic heterocyclic group include a five- or six-membered monocyclic aliphatic heterocyclic group containing at least one atom selected from a nitrogen atom, an oxygen atom, and a sulfur atom; and a bicyclic or tricyclic condensed aliphatic heterocyclic group which is formed by condensation of three- to eight-membered rings and contains at least one atom selected from a nitrogen atom, an oxygen atom, and a sulfur atom. In particular, pyrrolidinyl, 2,5-dioxopyrrolidinyl, thiazolidinyl, oxazolidinyl, piperidyl, piperidino, piperazinyl, homopiperazinyl, homopiperidyl, homopiperidino, morpholinyl, morpholino, thiomorpholinyl, thiomorpholino, pyranyl, tetrahydropyridyl, tetrahydropyranyl, tetrahydrofuranyl, tetrahydroquinolyl, tetrahydroisoquinolyl, octahydroquinolyl, indolinyl, and the like are included.
   (l) Halogen includes a fluorine atom, a chlorine atom, a bromine atom, and an iodine atom;
   (m) Examples of the condensed ring which is formed with the two adjacent carbon atoms and the carbon atom between these adjacent carbon atoms include a bicyclic to nona-cyclic fused ring having 6 to 60 carbon atoms, preferably 6 to 30 carbon atoms, which is formed by condensation of three- to eight-membered rings, wherein each ring may be saturated or unsaturated and may include an atom such as a nitrogen atom, an oxygen atom, and a sulfur atom, i.e. pyrene; cyclopentanophenanthrene; dibenzopentaphene; violanthrene; and a compound having a steroid skeleton including gonane, pregnane, estrane, cholestane, androstane, estratriene such as estra-1,3,5(10)-triene, androstene such as androst-4-ene, cholestene such as pregna-4-ene, estrapentaene, cholest-4-ene, cholest-5-ene, cholest-7-ene, and cholestadiene such as cholesta-3,5-diene; and
   (n) Examples of the carbocycle formed with the two carbon atoms which are adjacent to each groups, respectively, and the carbon atom between these two carbon atoms include cycloalkane, cycloalkene, cycloalkyne, cycloalkadiene, and the like.
      Examples of the cycloalkane include cycloalkane having 3 to 8 carbon atoms, i.e. cyclopropane, cyclobutane, cyclopentane, cyclohexane, cycloheptane, and cyclooctane. Examples of cycloalkene include cycloalkene having 4 to 8 carbon atoms, i.e. cyclobutene, cyclopentene, cyclohexene, cycloheptene, and cyclooctene. Examples of cycloalkyne include cycloalkyne having 4 to 8 carbon atoms, i.e. cyclobutyne, cyclopentyne, cyclohexyne, cycloheptyne, and cyclooctyne. Examples of cycloalkadiene include cycloalkadiene having 4 to 8 carbon atoms, i.e. cyclobutadiene, cyclopentadiene, cyclohexadiene, cycloheptadiene, and cyclooctadiene.
   (o) Examples of the aliphatic heterocycle which is formed with the two carbon atoms which are adjacent to each group, respectively, and the carbon atom between these two carbon atoms include a five- or six-membered monocyclic aliphatic heterocyclic group containing at least one atom selected from a nitrogen atom, an oxygen atom, and a sulfur atom; and a bicyclic or tricyclic condensed aliphatic heterocyclic group which is formed by condensation of three- to eight-membered rings and includes at least one atom selected from a nitrogen atom, an oxygen atom, and a sulfur atom; i.e. pyrrolidine, isothiazolidine, isooxazolidine, pyrazolidine, piperidine, homopiperazine, homopiperidine, tetrahydropyridine, tetrahydropyran, tetrahydrofuran, tetrahydroquinoline, tetrahydroisoquinoline, octahydroquinoline, and indoline; and
   (p) Substituents in the substituted lower alkyl, substituted lower alkoxy, substituted lower alkoxycarbonyl, substituted lower alkanoyl, and substituted lower alkanoyloxy may be the same or different, and the number of the substituents may be one to a substitutable number (the substitutable number is determined depending on the structure of a compound), preferably one to three, and examples of the substituents include substituted or unsubstituted lower alkoxy, substituted or unsubstituted lower alkoxycarbonyl, substituted or unsubstituted lower alkanoyl, substituted or unsubstituted lower alkanoyloxy, cycloalkyl, halogen, nitro, nitroso, carboxy, hydroxy, oxo, and a substituted or unsubstituted heterocyclic group. Also two substituents at two adjoining carbon atoms or at one carbon atom are combined together with the adjacent carbon atom(s) to form a ring such as substituted or unsubstituted carbocycle or substituted or unsubstituted aromatic carbocycle. Herein, the position of the substitution is not particulary limited.
      The lower alkyl moieties of the lower alkoxy, lower alkoxycarbonyl, lower alkanoyl and lower alkanoyloxy, cycloalkyl, halogen, and heterocyclic group shown here have the same meaning as lower alkyl (a), cycloalkyl (e), halogen (l), and heterocyclic group (k) above defined, respectively.
      The carbocycle formed by the two substituents and the two adjoining carbon atoms or the one carbon atom shown here have the same meaning as carbocycle (n) above defined, which is formed by the two adjacent carbon atoms and the carbon atom between these carbon atoms. Examples of the aromatic carbocycle include benzene, naphthalene, anthracene, indane, and the like.
      Substituents in the substituted lower alkoxy, substituted lower alkoxycarbonyl, substituted lower alkanoyl, substituted lower alkanoyloxy, substituted heterocyclic group, substituted carbocycle, and substituted aromatic carbocycle shown here may be the same or different. The number of the substituents may be one to three, and examples of the substituents include halogen (halogen has the same meaning as halogen (1) above defined), lower alkoxy (the lower alkyl moiety of the lower alkoxy has the same meaning as lower alkyl (a) above defined), and lower alkyl (lower alkyl has the same meaning as lower alkyl (a) defined above), and the like.
   (q) Substituents in the substituted lower alkenyl, substituted lower alkynyl, substituted lower alkadienyl, substituted cycloalkyl, substituted cycloalkenyl, substituted cycloalkynyl, substituted cycloalkadienyl, substituted aryl, substituted aryloxy, substituted aryloxycarbonyl, substituted aralkyl, substituted aralkyloxy, substituted aralkyloxycarbonyl, substituted heterocyclic group, substituted carbocycle which is formed by the two adjacent carbon atoms thereto and the carbon atom between these adjacent carbon atoms, substituted aliphatic heterocycle which is formed with the two adjacent carbon atoms thereto and the carbon atom between these adjacent carbon atoms, and substituted condensed ring which is formed with the two adjacent carbon atoms thereto and the carbon atom between these adjacent carbon atoms may be the same or different, and the number of the substituents may be one to a substitutable number (the substitutable number is determined depending on the structure of a compound), and examples of the substituents include the groups described in (p) as the substituents of substituted lower alkyl and also include substituted or unsubstituted lower alkyl, substituted or unsubstituted aryl, and a substituted or unsubstituted heterocyclic group. Herein, the position of the substitution is not particulary limited.
      The lower alkyl, aryl, and heterocyclic group shown here have the same meaning as the lower alkyl (a), aryl (j), and heterocyclic group (k) above defined, respectively.
      The substituents in the substituted lower alkyl, substituted aryl, and substituted heterocyclic group are the same as substituents (p) in the substituted lower alkyl above defined.
      In the present invention:
   (r) Examples of the acid include hydrochloric acid, sulfuric acid, nitric acid, acetic acid, trifluoroacetic acid, trifluoromethanesulfonic acid, methanesulfonic acid, and the like;
   (s) Examples of the acid anhydride include acetic anhydride, trifluoroacetic anhydride, trifluoromethanesulfonic anhydride, methanesulfonic anhydride, and the like; and
   (t) A suspension or a solution containing Compound (II) and Compound (III) is prepared by suspending or dissolving Compound (II) and Compound (III) in, for example, pentene, methylene chloride, chloroform, carbon tetrachloride, 1,2-dichloroethane, chlorobenzene, dichlorobenzene, N-methylpiperidone, ethyl acetate, toluene, trifluorotoluene, and the like, or a mixture thereof. Among them, a solution or suspension of methylene chloride, chlorobenzene, dichlorobenzene, toluene, and trifluorotoluene is preferable.

   Examples of the process for producing Compound (IV) will be described below.

### Process 1:

### Compound (IV) is produced according to the following steps:

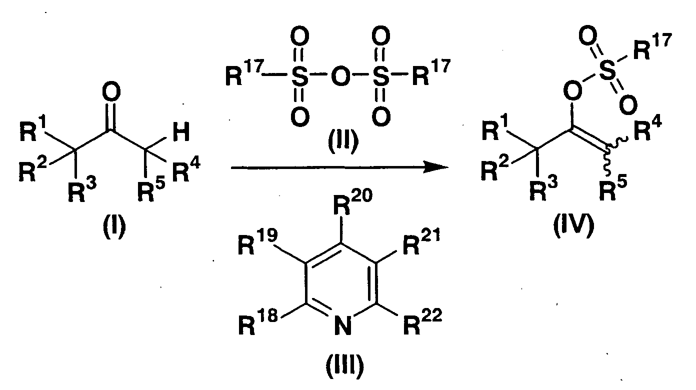

(wherein R¹, R², R³, R⁴, R⁵, R¹⁷, R¹⁸, R¹⁹, R²⁰, R²¹ and R²² have the same meanings as defined above)

Compound (IV) is obtained by dissolving or suspending Compound (I) in a solvent in the presence of Compound (III) in an amount of 0.1 to 1.0 equivalent, preferably 0.3 to 1.0 equivalent, more preferably 0.8 to 1.0 equivalent to Compound (II), and adding Compound (II) to the resulting solution or suspension which is stirred to react. Compound (II) may be added all at once or dropwise according to the progress of reaction.

The amount of Compound (II) is preferably 1.0 to 5.0 equivalent, more preferably 1.0 to 2.5 equivalent, most preferably 1.0 to 1.5 equivalent to Compound (I).

During the reaction, Compound (II), water, an acid, an acid anhydride or the like may be further added in an amount of preferably 0.005 to 1.0 equivalent, more preferably 0.01 to 0.5 equivalent to Compound (I). Examples of the acid include hydrochloric acid, sulfuric acid, nitric acid, acetic acid, trifluoroacetic acid, trifluoromethanesulfonic acid, and methanesulfonic acid. Examples of the acid anhydride include acetic anhydride, trifluoroacetic anhydride, trifluoromethanesulfonic anhydride, methanesulfonic anhydride, and the like. Supplemental addition may be appropriately performed several times according to the progress of reaction.

Examples of the solvent include pentene, methylene chloride, chloroform, carbon tetrachloride, 1,2-dichloroethane, chlorobenzene, dichlorobenzene, N-methylpiperidone, ethyl acetate, toluene, trifluorotoluene, and the like, and these are used alone or as a mixture thereof. Among them, methylene chloride, chlorobenzene, dichlorobenzene, toluene, trifluorotoluene, and the like are preferable.

The reaction is performed at a temperature between 0°C and the boiling point of the solvent used, preferably at a temperature between 15°C and 50°C, usually for 2 to 72 hours.

### Process 2:

### Compound (IV) can be produced according to the following steps:

Compound (IV) is obtained by adding Compound (I) to a suspension or a solution containing Compound (II) and Compound (III) which is stirred to react, wherein Compound (III) is contained in an amount of 0.1 to 1.0 equivalent to Compound (II). When the amount of Compound (III) is 1.0 equivalent to Compound (II), Compound (II), water, an acid, or an acid anhydride is further added. Compound (I) may be added all at once, gradually, or dropwise according to the progress of reaction.

The amount of Compound (II) is preferably 1.0 to 5.0 equivalent, more preferably 1.0 to 2.5 equivalent, most preferably 1.0 to 1.5 equivalent to Compound (I).

The amount of Compound (III) is preferably 0.1 to 1.0 equivalent, more preferably 0.3 to 1.0 equivalent, most preferably 0.8 to 1.0 equivalent to Compound (II).

During the reaction, Compound (II), water, an acid, an acid anhydride or the like may be further added in an amount of preferably 0.005 to 1.0 equivalent, more preferably 0.01 to 0.5 equivalent to Compound (I). Examples of the acid include hydrochloric acid, sulfuric acid, nitric acid, acetic acid, trifluoroacetic acid, trifluoromethanesulfonic acid, and methanesulfonic acid. Examples of the acid anhydride include acetic anhydride, trifluoroacetic anhydride, trifluoromethanesulfonic anhydride, and methanesulfonic anhydride. Supplemental addition may be appropriately performed several times according to the progress of reaction.

The suspension or solution containing Compound (II) and Compound (III) is prepared by suspending or dissolving Compound (II) and Compound (III) in, for example, pentene, methylene chloride, chloroform, carbon tetrachloride, 1,2-dichloroethane, chlorobenzene, dichlorobenzene, N-methylpiperidone, ethyl acetate, toluene, trifluorotoluene, or a mixture thereof. Among them, a solution or suspension of methylene chloride, chlorobenzene, dichlorobenzene, toluene, or trifluorotoluene is preferable.

When Compound (I) is added, Compound (I) may be dissolved in a solvent if necessary, and then added. Examples of the solvent include pentene, methylene chloride, chloroform, carbon tetrachloride, 1,2-dichloroethane, chlorobenzene, dichlorobenzene, N-methylpiperidone, ethyl acetate, toluene, and trifluorotoluene. These are used alone or as a mixture thereof. Among them, methylene chloride, chlorobenzene, dichlorobenzene, toluene, trifluorotoluene and the like are preferable.

The reaction is performed at a temperature between 0°C and the boiling point of the solvent, preferably at a temperature between 15°C and 50°C, usually for 2 to 72 hours.

### Process 3:

### Compound (IV) can be produced according to the following steps:

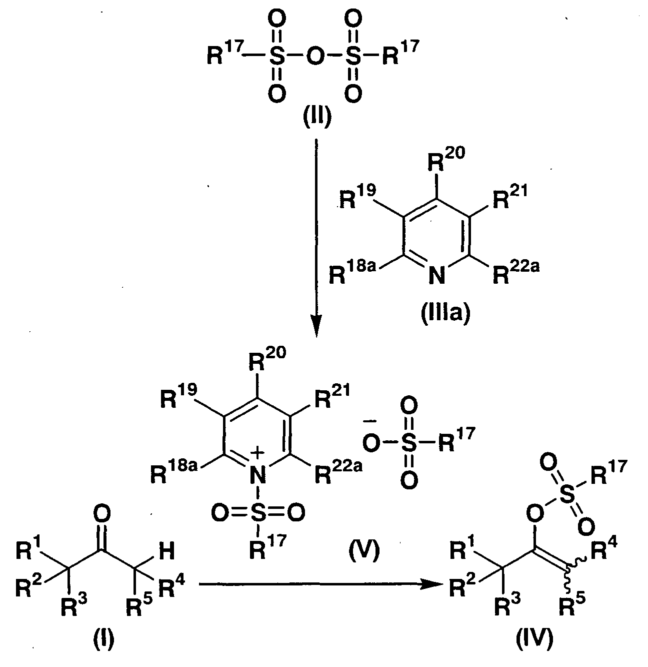

(wherein R¹, R², R³, R⁴, R⁵, R¹⁷, R^{18a}, R¹⁹, R²⁰, R²¹ and R^{22a} have the same meanings as defined above)

### Step 1: Preparation of Compound (V)

Compound (II) is dissolved or suspended in a solvent. Compound (IIIa) is added to the obtained solution or suspension under cooling, and then the solution or the suspension is stirred at a temperature between 0°C and 40°C for 15 minutes to 2 hours to prepare a solution or suspension of Compound (V). The obtained Compound (V) can be used in the next step as the solution or the suspension without purification or as a solid obtained by concentration, filtration, or the like.

The amount of Compound (IIIa) is 0.1 to 1.0 equivalent, preferably 0.3 to 1.0 equivalent, more preferably 0.8 to 1.0 equivalent to Compound (II).

Examples of the solvent include pentene, methylene chloride, chloroform, carbon tetrachloride, 1,2-dichloroethane, chlorobenzene, dichlorobenzene, N-methylpiperidone, ethyl acetate, toluene, trifluorotoluene, and the like. The solvents may be used alone or as a mixture thereof. Among them, methylene chloride, chlorobenzene, dichlorobenzene, toluene, trifluorotoluene, and the like are preferable.

### Step 2:

Compound (IV) can be obtained by reacting Compound (I) with Compound (V) prepared in the step 1 in a solvent. In this step, Compound (V) may be added to a solution or a suspension of Compound (I), or Compound (I) may be added to a solution or a suspension of Compound (V). When Compound (V) or Compound (I) is added, each compound may be added all at once, or may be dissolved or suspended in a solvent and then added dropwise according to the progress of reaction.

The amount of Compound (V) is preferably 1.0 to 5.0 equivalent, more preferably 1.0 to 3.0 equivalent to Compound (I).

After the addition of Compound (V) or Compound (I), Compound (II), water, an acid, or an acid anhydride may be appropriately added in an amount of preferably 0.005 to 1.0 equivalent, more preferably 0.01 to 0.5 equivalent to Compound (I) during the reaction. Examples of the acid include hydrochloric acid, sulfuric acid, nitric acid, acetic acid, trifluoroacetic acid, trifluoromethanesulfonic acid, methanesulfonic acid, and the like. Examples of the acid anhydride include acetic anhydride, trifluoroacetic anhydride, trifluoromethanesulfonic anhydride, and methanesulfonic anhydride. Supplemental addition may be appropriately performed several times according to the progress of reaction.

Examples of the solvent include pentene, methylene chloride, chloroform, carbon tetrachloride, 1,2-dichloroethane, chlorobenzene, dichlorobenzene, N-methylpiperidone, ethyl acetate, toluene, trifluorotoluene, and the like, and these solvents may be used alone or as a mixture thereof. Among them, methylene chloride, chlorobenzene, dichlorobenzene, toluene, trifluorotoluene, and the like are preferable.

The reaction is performed at a temperature between 0°C and the boiling point of the solvent, preferably at a temperature between 15°C and 50°C, usually for 2 to 72 hours

In each process described above, it is preferable to perform the reaction under argon or nitrogen atmosphere. However, it is possible to perform the reaction in the atmosphere.

Compound (I) as starting material is commercially available or can be prepared by a known method for synthesis of carbonyl compounds (See, for example, Shin Jikken Kagaku Kouza (New course of Chemical Experiment) 14 "Synthesis and reaction of organic compound II", p. 751, published by Maruzen (1977); Jikken Kagaku Kouza (Course of Chemical Experiment), 4th edition, vol. 21 "Organic synthesis III", p, 149, published by Maruzen (1991); Organic Functional Group Preparations, second edition, written by S. R. Sandler and W. Karo, published by Academic Press (1989); Survey of Organic Synthesis, vol. 2, written by C. A. Buehler and D. E. Pearson, published by Wiley-Interscience Publication John Wiely & Sons (1992)).

In each process described above, when the defined groups in Compound (I) are changed under the reaction condition or are unsuitable for the process, Compound (I) are applied to any known methods in the field of synthetic organic chemistry, for example, protection and deprotection of the functional groups, so that the process can be readily conducted (See, for example, Protective Groups in Organic Synthesis, third edition, written by T. W. Greene, published by John Wiley & Sons Inc.(1999)). In each process described above, when the defined groups in Compound (I) have a nitrogen atom which may form a salt together with Compound (II), it is preferable that a salt formed by the nitrogen atom and, for example, trifluoromethanesulfonic acid, methanesulfonic acid, or an acid anhydride thereof, be used as a starting material in the process.

Compound (IV) and Compound (V) obtained in the aforementioned process can be isolated and purified by the methods generally used in the field of synthetic organic chemistry, for example, filtration, extraction, washing, drying, condensation, recrystallization, distillation, active carbon treatment, and various chromatography. Particularly, Compound (II), Compound (III) remaining after the reactions and the perfluoroalkanesulfonate of Compound (III) generated as a by-product can be easily removed by appropriate combination of filtration, extraction, washing, and the like.

Compound (V) can be applied to the aforementioned process without particular purification. Compound (IV) can be applied to the following reaction as the intermediates of medicines and natural products without further purification.

Some of starting materials or intermediates in the production methods may be in the form of salts depending on the reaction condition or the like, for example, trifluoromethanesulfonate, hydrochloride, sodium salt, and potassium salt. These compounds can be used as they are, or used in the free form. For using or preparing these starting materials or intermediates in the form of salts, the starting materials or intermediates are used without modification when they are obtained as salts. For using or preparing the starting materials or intermediates in the free form, the starting materials or intermediates can be converted into free form by being dissolved or suspended into an appropriate solvent, and then being neutralized with a base such as sodium hydrogen carbonate solution or an acid such as hydrochloric acid or acetic acid.

Some of Compound (I) and Compound (IV) may have an isomer such as a regio isomer, a geometrical isomer, or an optical isomer, and all possible isomers including these isomers and mixtures in any ratio thereof can be used as a starting material in the present invention, or can be produced in the present invention.

For preparing a salt of Compound (IV), when Compound (IV) is produced in the form of a salt, Compound (IV) is purified as it is; and when Compound (IV) is produced in the free form, Compound (IV) is dissolved or suspended to an appropriate solvent and then an acid or a base is added to form the salt.

Compound (IV) may be present in the form of adducts with water or various solvents. These adducts can be produced by a process according to the present invention.

Examples of Compound (IV) produced according to the present invention are shown in Table 1.

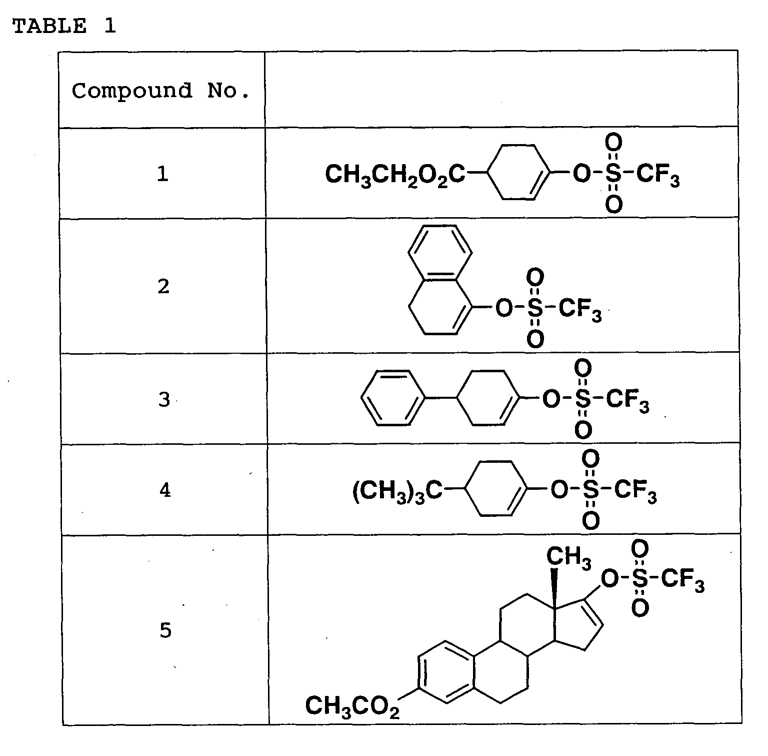

Compound (IV) produced by the processes according to the present invention is a useful intermediate in synthesis of medicines, natural products, and the like, and is used in for example, Suzuki-Miyaura reaction, cross-coupling reaction, carbon-carbon bond formation, CO-introducing reaction, or Nozaki-Hiyama reaction (See, for example, Synthesis, p. 735 (1993); Tetrahedron Lett., vol. 23, p. 117 (1982); ibid., vol. 24, p. 979 (1983); J. Org. Chem., vol. 56, p. 3486 (1991); ibid., vol. 57, p. 976 (1992); ibid., vol. 63, p. 4135 (1998); Synthetic Communications, vol. 29, p. 409 (1999)).

### Best Mode for Carrying Out the Invention

The present invention will now be described in detail with referring to EXAMPLES and REFERENCE EXAMPLES. However, the scope of the present invention is not limited to EXAMPLES and REFERENCE EXAMPLES.

### EXAMPLE 1: Synthesis of vinyl trifluoroalkanesulfonate ester derivatives (Compound (IV))

Pyridine (142 mg, 1.80 mmol) was dissolved in toluene or methylene chloride (5.0 mL), and then trifluoromethanesulfonic anhydride (550 mg, 1.95 mmol) was added. The solution was stirred at 25°C for 30 minutes. Then corresponding carbonyl compound (Compound (I)) (1.50 mmol) was added and the solution was stirred for 2 to 27 hours. After acetonitrile was added, the yields of the vinyl trifluoroalkanesulfonate derivatives were determined by high performance liquid chromatographic (HPLC) analysis or gas chromatographic (GLC) analysis. The determined yields are shown in Table 2. The measurement conditions of the HPLC and the GLC are as follows:
HPLC conditions (for measuring Compounds 2, 3, and 5)
Apparatus: Hitachi, Ltd.
Column: Cadenza CD-C-18, 75 mm × 4.6 mm (Imtakt Corporation)
Mobile phase: CH₃OH:0.01 mol/L KH₂PO₄ solution = 2:1
Temperature: 35°C
Flow rate: 0.8 mL/min
Detection: UV at 215 nm
GLC conditions (for measuring Compounds 1 and 4)
Apparatus: Shimadzu Corporation
Column: TC-5, 30 m × 0.25 mm, I.D. 0.25 µm (GL Sciences Inc.)
Carrier gas: He
Temperature: Injection at 250°C, Detection at 260°C
   Column initial temperature; 100°C (5 min)
   Column heating rate; 5°C/min (after 5 min)
   Column final temperature; 250°C
Detection: FID
Injection: Split method

**TABLE 2**

| Compound No. | Solvent | Reaction Temperature (°C) | Yield (%) |
|---|---|---|---|
| 1 | Methylene chloride | 40 | 90 |
| | Toluene | 40 | 91 |
| 2 | Methylene chloride | 25 | 91 |
| | Toluene | 25 | 88 |
| 3 | Methylene chloride | 25 | 80 |
| 4 | Methylene chloride | 25 | 60 |
| 5 | Methylene chloride | 25 | 91 |
| | Toluene | 25 | 97 |

### EXAMPLE 2: Synthesis of vinyl trifluoroalkanesulfonate ester derivatives (Compound 2)

Pyridine (154 mg, 1.95 mmol) was dissolved in toluene or methylene chloride (5.0 mL), and then trifluoromethanesulfonic anhydride (550 mg, 1.95 mmol) was added at 25°C and stirred for 30 minutes. Then α-tetralone (219 mg, 1.50 mmol) and an additive (0.15 mmol) shown in Table 3 were added and the solution was stirred at 25°C for 1 to 30 hours. After acetonitrile was added, and the yields of the vinyl trifluoroalkanesulfonate ester derivatives were determined by HPLC analysis. The determined yields are shown in Table 3. The measurement conditions of the HPLC were the same as those in EXAMPLE 1.

**TABLE 3**

| Compound No. | Additive | Solvent | Yield (%) |
|---|---|---|---|
| 2 | Water | Methylene chloride | 87 |
| | Trifluoromethanesulfonic anhydride | Methylene chloride | 91 |
| | Trifluoromethanesulfonic acid | Methylene chloride | 90 |
| | | Toluene | 89 |
| | Methanesulfonic acid | Methylene chloride | 84 |
| | Acetic acid | Methylene chloride | 86 |
| | | Toluene | 77 |

### EXAMPLE 3: Synthesis of 4-ethoxycarbonylcyclohexen-1-yl trifluoromethanesulfonate (Compound 1)

Pyridine (10.19 g) was dissolved in toluene (350 mL), and trifluoromethanesulfonic anhydride (36.68 g) was added. The solution was stirred at 15°C for 30 minutes. Then 4-ethoxycarbonylcyclohexanone (20.00 g) was added and the solution was stirred at 40°C. Thereafter, trifluoromethanesulfonic anhydride (0.17 g) was added after 10 hours and 12 hours. After stirring for 2 hours, water was added to the resulting reaction mixture, the mixture was stirred for 30 minutes, and then separated into layers. To the organic layer, silica gel (60 g) was added and the mixture was stirred at room temperature for 30 minutes. The silica gel was removed by filtration, and then the solvent was evaporated to give a yellow liquid of Compound 1 (31.6 g, a yield of 89%) was obtained.

GLC analysis (the measurement conditions were the same as those in EXAMPLE 1) and ¹H nuclear magnetic resonance (NMR) analysis indicated that pyridine did not remain in the organic layer and in the yellow liquid of Compound 1.
¹H-NMR (CDCl₃, δ, ppm) 1.27 (t, J=7.1 Hz, 3H), 1.86-1.99 (m, 1H), 2.09-2.18 (m, 1H), 2.38-2.48 (m, 4H), 2.53-2.64 (m, 1H), 4.16 (q, J=7.1 Hz, 2H), 5.77-5.79 (m, 1H).
MASS ESI(+) (m/z) 303 (M+H).

### EXAMPLE 4: Synthesis of 1,2-dihydronaphthalen-4-yl trifluoromethanesulfonate (Compound 2)

Pyridine (3.56 g) was dissolved in toluene (100 mL), and then trifluoromethanesulfonic anhydride (13.75 g) was added. The mixture was stirred at 25°C for one hour. To the reaction mixture, α-tetralone (5.48 g) was added and the mixture was stirred at 25°C for 20 hours. Then 2% sodium hydrogen carbonate solution was added. The separated organic layer was washed with 1 mol/L hydrochloric acid, then with 5% saline. Activated carbon (0.50 g) was added and the resulting mixture was stirred at room temperature for one hour. The activated carbon was removed by filtration, and then the solvent was evaporated to give a light brown liquid of Compound 2 (10.08 g, 97%).
¹H-NMR (CDCl₃, δ, ppm) 2.46-2.53 (m, 2H), 2.86 (t, J=8.2 Hz, 2H), 6.00 (t, J=4.8 Hz, 1H), 6.88-7.19 (m, 1H), 7.21-7.29 (m, 2H), 7.32-7.37 (m, 1H).
MASS ESI(+) (m/z) 279(M+H).

### EXAMPLE 5: Synthesis of 3-acetoxy-1,3,5(10),16-estratetraen-17-yl trifluoromethanesulfonate (Compound 5)

17-Oxo-1,3,5(10)-estratrien-3-yl acetate (5.90 g) was dissolved in toluene (59 mL), and then pyridine (1.80 g) and trifluoromethanesulfonic anhydride (6.94 g) were added. The mixture was stirred at 25°C for 26 hours. After the addition of water, the reaction mixture was extracted with ethyl acetate. The extracted solution was washed with 5% sodium hydrogen carbonate, then with 1 mol/L hydrochloric acid and 5% saline. Activated carbon (0.59 g) was added and the mixture was stirred at room temperature for 30 minutes. The activated carbon was removed by filtration, and then the solvent was evaporated to give the crude crystalline product of Compound 5 (8.12 g, 97%). The obtained crude crystalline product was recrystallized from heptane (30 mL) to give Compound 5 (6.70 g, 80%) as a colorless crystal.
Melting point: 68°C (decomposed)
¹H-NMR (CDCl₃, δ, ppm) 1.00 (s, 3H), 1.38-1.67 (m, 4H), 1.79 (dt, J=6.2 Hz, 11.0 Hz, 1H), 1.88-1.96 (m, 2H), 2.10 (ddd, J=1.6 Hz, 11.0 Hz, 14.9 Hz, 1H), 2.28 (s, 3H), 2.28-2.50 (m, 3H), 2.88-2.92 (m, 2H), 5.62 (dd, J=1.6 Hz, 3.3 Hz, 1H), 6.81 (d, J=2.6 Hz, 1H), 6.85 (dd, J=2.6 Hz, 8.4 Hz, 1H), 7.25 (d, J=8.4 Hz, 1H).
MASS ESI(+) (m/z) 445(M+H).

### COMPARATIVE EXAMPLE 1: Synthesis of 4-ethoxycarbonylcyclohexen-1-yl trifluoromethanesulfonate (Compound 1) using 2,6-di-tert-butyl-4-methylpyridine

4-Ethoxycarbonylcyclohexanone (1.00 g) was dissolved in methylene chloride (20 mL), and then 2,6-di-tert-butyl-4-methylpyridine (1.81 g, 8.81 mmol) was added. To the solution, trifluoromethanesulfonic anhydride (2.15 g, 7.63 mmol) was added at 15°C and the mixture was stirred under reflux for 3 hours. After the reaction (the yield was 82.7%), the reaction solution was cooled with ice, and precipitated insolubles (2,6-di-tert-butyl-4-methylpyridine and trifluoromethanesulfonate thereof) were removed by filtration (purification 1). The same step was further repeated twice and then the filtrate was washed with water and a sodium hydrogen carbonate solution (purification 2). The resulting solution was purified by silica-gel column chromatography to give Compound 1 (1.16 g, a yield of 65.3%) as a yellow liquid. GLC analysis (the measurement conditions of GLC were the same as those in EXAMPLE 1) indicated that a large amount of 2,6-di-*tert*-butyl-4-methylpyridine remained in the solutions after purification 1 and 2. In regard with the yellow liquid of Compound 1, ¹H-NMR analysis indicated that a trace amount of 2,6-di-*tert*-butyl-4-methylpyridine remained.

### COMPARATIVE EXAMPLE 2: Synthesis of 4-ethoxycarbonylcyclohexen-1-yl trifluoromethanesulfonate (Compound 1) using pyridine

4-Ethoxycarbonylcyclohexanone (100 mg) was dissolved in methylene chloride (2 mL), and then pyridine (69.7 mg, 0.88 mmol) was added. To the resulting solution, trifluoromethanesulfonic anhydride (199 mg, 0.70 mmol) was added at 15°C and the mixture was stirred under reflux for 4 hours. Then acetonitrile was added. The yield of 4-ethoxycarbonylcyclohexanone determined by GLC analysis was 0.1%. The measurement conditions of the GLC analysis were the same as those in EXAMPLE 1.

### COMPARATIVE EXAMPLE 3: Synthesis of a vinyl trifluoroalkanesulfonate derivative (Compound 2)

Pyridine (154mg, 1.95 mmol) was dissolved in toluene (5.0 mL), and then trifluoromethanesulfonic anhydride (550 mg, 1.95 mmol) was added. The solution was stirred at 25°C for 30 minutes. Then α-tetralone (219 mg, 1.50 mmol) was added and the mixture was stirred at 25°C for 30 hours. After the reaction, acetonitrile was added. The yield of the vinyl trifluoroalkanesulfonate derivative was determined by HPLC analysis (the yield was 0.4%). The measurement conditions of the HPLC were the same as those in EXAMPLE 1.

### Industrial Applicability

The present invention provides a simple and easy process for producing a vinyl perfluoroalkanesulfonate derivative useful as an intermediate in synthesis of medicines, natural products, or the like, at a low cost by using reagents that are inexpensive and readily available in large quantity.

## Claims

1. A process for producing a vinyl perfluoroalkanesulfonate derivative represented by general formula (IV): 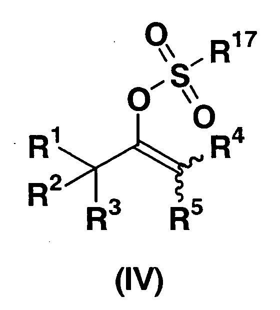 (wherein R¹, R², R³, R⁴ and R⁵ may be the same or different and each represents a hydrogen atom, substituted or unsubstituted lower alkyl, substituted or unsubstituted lower alkoxy, substituted or unsubstituted lower alkoxycarbonyl, substituted or unsubstituted lower alkanoyl, substituted or unsubstituted lower alkanoyloxy, substituted or unsubstituted lower alkenyl, substituted or unsubstituted lower alkynyl, substituted or unsubstituted lower alkadienyl, substituted or unsubstituted cycloalkyl, substituted or unsubstituted cycloalkenyl, substituted or unsubstituted cycloalkynyl, substituted or unsubstituted cycloalkadienyl, substituted or unsubstituted aralkyl, substituted or unsubstituted aralkyloxy, substituted or unsubstituted aralkyloxycarbonyl, substituted or unsubstituted aryl, substituted or unsubstituted aryloxy, substituted or unsubstituted aryloxycarbonyl, a substituted or unsubstituted heterocyclic group, nitro, nitroso, halogen, carboxy, -S(O)ₙR⁶ (wherein n represents 0 or 1, and R⁶ represents substituted or unsubstituted lower alkyl, substituted or unsubstituted aralkyl, or substituted or unsubstituted aryl group), -P(O)ₘR^{6a}R^{6b} (wherein m represents 0 or 1, and R^{6a} and R^{6b} may be the same or different and each has the same meaning as R⁶ defined above), or -NR⁷R⁸ [wherein R⁷ and R⁸ may be the same or different and each represents a hydrogen atom, substituted or unsubstituted lower alkyl, substituted or unsubstituted aralkyl, substituted or unsubstituted aryl, substituted or unsubstituted lower alkoxycarbonyl, substituted or unsubstituted lower alkanoyl, substituted or unsubstituted lower alkanoyloxy, -CONR^{6c}R^{6d} (wherein R^{6c} and R^{6d} may be the same or different and each has the same meaning as R⁶ defined above), or -SO₂R^{6e} (wherein R^{6e} has the same meaning as R⁶ defined above)];
R¹ and R² are combined together with the adjacent carbon atom thereto to form R⁹ (wherein R⁹ represents substituted or unsubstituted cycloalkyl, substituted or unsubstituted cycloalkenyl, substituted or unsubstituted cycloalkynyl, substituted or unsubstituted cycloalkadienyl, or a substituted or unsubstituted heterocyclic group);
R⁴ and R⁵ are combined together with the adjacent carbon atom thereto to form R¹⁰ (wherein R¹⁰ has the same meaning as R⁹ defined above);
R¹, R² and R³ are combined together with the adjacent carbon atom thereto to form R¹¹ (wherein R¹¹ represents substituted or unsubstituted aryl, or a substituted or unsubstituted heterocyclic group);
R¹ and R⁴ are combined together with the two carbon atoms which are adjacent to R¹ or R⁴, respectively, and the carbon atom between these two carbon atoms to form a substituted or unsubstituted carbocycle, or a substituted or unsubstituted aliphatic heterocycle; or
R¹, R², R³, R⁴ and R⁵ are combined together with the two carbon atoms which are adjacent to R¹, R², R³, R⁴ or R⁵, respectively, and the carbon atom between these two carbon atoms to form a substituted or unsubstituted carbocycle, a substituted or unsubstituted aliphatic heterocycle, or a substituted or unsubstituted condensed ring, and
R¹⁷ represents a fluorine atom or perfluoroalkyl)
which comprises reacting a carbonyl compound represented by general formula (I): 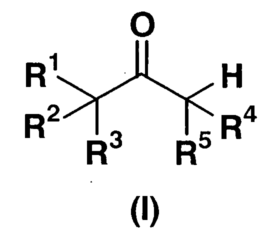 (wherein R¹, R², R³, R⁴ and R⁵ have the same meanings as defined above, respectively)
with a perfluoroalkanesulfonic anhydride represented by general formula (II): 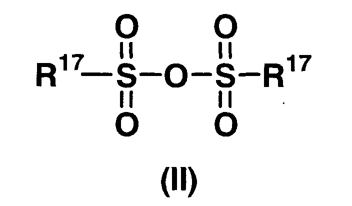 (wherein R¹⁷ has the same meaning as defined above)
in the presence of a pyridine derivative represented by general formula (III) in an amount of 0.1 to 1.0 equivalent to the perfluoroalkanesulfonic anhydride: 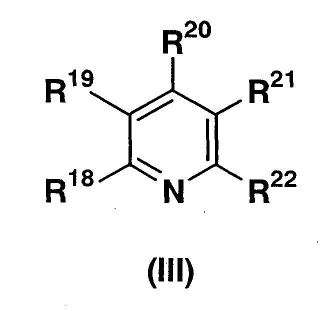 (wherein R¹⁸, R¹⁹, R²⁰, R²¹ and R²² may be the same or different and each represents a hydrogen atom, halogen, substituted or unsubstituted lower alkyl, or substituted or unsubstituted lower alkoxy; with the proviso that when R¹⁸ and R²² are tert-butyl, and R¹⁹ and R²¹ are hydrogen atoms, R²⁰ is not methyl).

2. A process for producing a vinyl perfluoroalkanesulfonate derivative represented by general formula (IV): 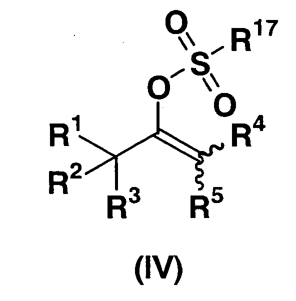 (wherein R¹, R², R³, R⁴, R⁵ and R¹⁷ have the same meanings as defined above, respectively)
which comprises adding a carbonyl compound represented by general formula (I): 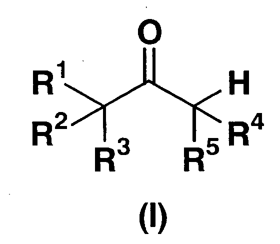 (wherein R¹, R², R³, R⁴ and R⁵ have the same meanings as defined above, respectively)
to a suspension or a solution containing a perfluoroalkanesulfonic anhydride represented by general formula (II): 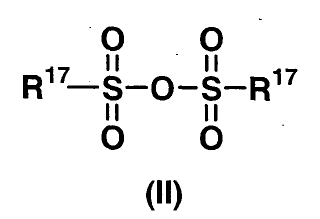 (wherein R¹⁷ has the same meaning as defined above)
and a pyridine derivative represented by general formula (III) in an amount of 0.1 to 1.0 equivalent to the perfluoroalkanesulfonic anhydride: 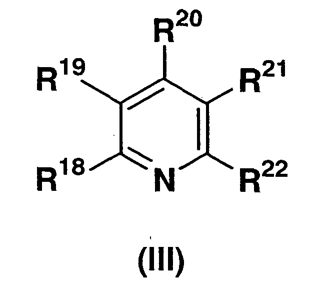 (wherein R¹⁸, R¹⁹, R²⁰, R²¹ and R²² have the same meanings as defined above, respectively), and wherein
when the content of the pyridine derivative represented by general formula (III) in the suspension or the solution is 1.0 equivalent to the perfluoroalkanesulfonic anhydride represented by general formula (II), the perfluoroalkanesulfonic anhydride represented by general formula (II), water, an acid, or an acid anhydride is further added for the reaction.

3. A process for producing a vinyl perfluoroalkanesulfonate derivative represented by general formula (IV): 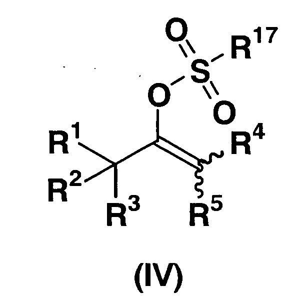 (wherein R¹, R², R³, R⁴, R⁵ and R¹⁷ have the same meanings as defined above, respectively)
which comprises reacting a carbonyl compound represented by general formula (I): 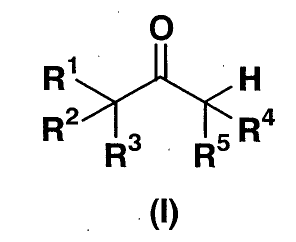 (wherein R¹, R², R³, R⁴ and R⁵ have the same meanings as defined above, respectively)
with a 1-(perfluoroalkanesulfonyl)pyridinium perfluoroalkanesulfonate represented by general formula (V): 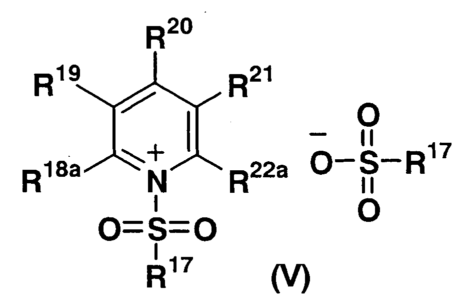 (wherein R¹⁹, R²⁰ and R²¹ have the same meanings as defined above, respectively, and R^{18a} and R^{22a} may be the same or different and each represents a hydrogen atom, methyl, ethyl, *n*-propyl, isopropyl, methoxy, ethoxy, *n*-propyloxy, or isopropyloxy).

4. A process for producing a vinyl perfluoroalkanesulfonate derivative represented by general formula (IV): 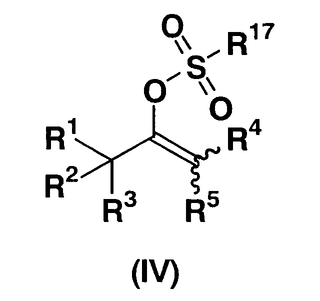 (wherein R¹, R², R³, R⁴, R⁵ and R¹⁷ have the same meanings as defined above, respectively)
which comprises preparing a 1-(perfluoroalkanesulfonyl)pyridinium perfluoroalkanesulfonate represented by general formula (V): 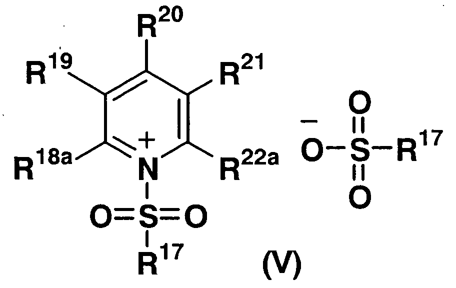 (wherein R^{18a}, R¹⁹, R²⁰, R²¹ and R^{22a} have the same meanings as defined above, respectively)
from a perfluoroalkanesulfonic anhydride represented by general formula (II): 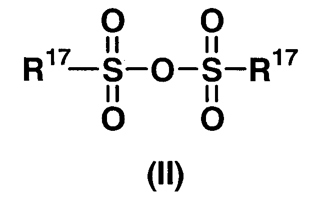 (wherein R¹⁷ has the same meanings as defined above)
and a pyridine derivative represented by general formula (IIIa): 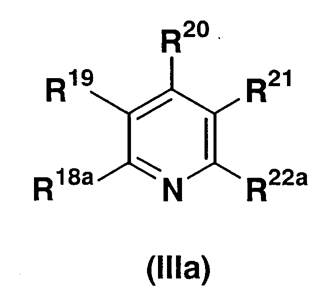 (wherein R^{18a}, R¹⁹, R²⁰, R²¹ and R^{22a} have the same meanings as defined above, respectively);
and then reacting the resulting 1-(perfluoroalkanesulfonyl)pyridinium perfluoroalkanesulfonate with a carbonyl compound represented by general formula (I): 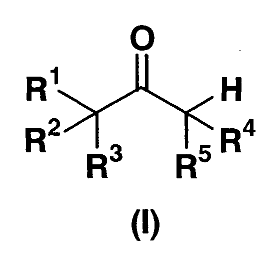 (wherein R¹, R², R³, R⁴, and R⁵ have the same meanings as defined above, respectively).

5. The process according to claim 1 or 2, wherein R¹⁸ and R²² may be the same or different and each represents a hydrogen atom, methyl, ethyl, *n*-propyl, isopropyl, methoxy, ethoxy, *n*-propyloxy, or isopropyloxy.

6. The process according to claim 1 or 2, wherein R¹⁸ and R²² may be the same or different and each represents a hydrogen atom, halogen, or methyl.

7. The process according to any one of claims 1 to 6, wherein R¹⁹ and R²¹ represent a hydrogen atom.

8. The process according to claim 7, wherein R²⁰ represents a hydrogen atom or methyl.

9. The process according to claim 1 or 2, wherein R¹⁸, R¹⁹, R²⁰, R²¹, and R²² represent a hydrogen atom.

10. The process according to claim 3 or 4, wherein R^{18a}, R¹⁹, R²⁰, R²¹, and R^{22a} represent a hydrogen atom.

11. The process according to any one of claims 1 to 10, wherein the perfluoroalkanesulfonic anhydride represented by general formula (II), water, an acid, or an acid anhydride is further added during the reaction of the perfluoroalkanesulfonic anhydride represented by general formula (II): 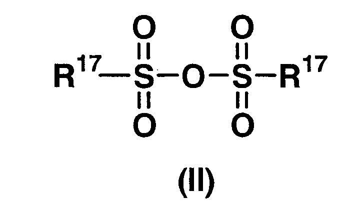 (wherein R¹⁷ has the same meaning as defined above).

12. The process according to any one of claims 1 to 11, wherein R¹⁷ represents a fluorine atom, trifluoromethyl, or nonafluoro-*n*-butyl.

13. The process according to any one of claims 1 to 11, wherein R¹⁷ represents a fluorine atom or trifluoromethyl.

14. The process for producing a vinyl perfluoroalkanesulfonate derivative according to any one of claims 1 to 13, wherein at least one selected from the group consisting of methylene chloride, toluene, chlorobenzene, trifluorotoluene, and dichlorobenzene is used as the solvent.

15. The process for producing a vinyl perfluoroalkanesulfonate derivative according to any one of claims 1 to 14, wherein R¹ and R⁴ are combined together with the two carbon atoms which are adjacent to R¹ or R⁴, respectively, and the carbon atom between these two carbon atoms to form a substituted or unsubstituted carbocycle, or a substituted or unsubstituted aliphatic heterocycle; or R¹, R², R³, R⁴ and R⁵ are combined together with the two carbon atoms which are adjacent to R¹, R², R³, R⁴ or R⁵, respectively, and the carbon atom between these two carbon atoms to form a substituted or unsubstituted carbocycle, a substituted or unsubstituted aliphatic heterocycle, or a substituted or unsubstituted condensed ring.

16. The process for producing a vinyl perfluoroalkanesulfonate derivative according to any one of claims 1 to 14, wherein R¹, R², R³, R⁴ and R⁵ are combined together with the two carbon atoms which are adjacent to R¹, R², R³, R⁴ or R⁵, respectively, and the carbon atom between these two carbon atoms to form a substituted or unsubstituted carbocycle.

17. The process for producing a vinyl perfluoroalkanesulfonate derivative according to any one of claims 1 to 14, wherein R¹, R², R³, R⁴ and R⁵ are combined together with the two carbon atoms which are adjacent to R¹, R², R³, R⁴ or R⁵, respectively, and the carbon atom between these two carbon atoms to form a substituted or unsubstituted condensed ring.
